# EUROPEAN PATENT APPLICATION

(11) **EP 2 609 814 A1**
(43) Date of publication of application: **03.07.2013**
(21) Application number: 11196139.7
(22) Date of filing: 30.12.2011
(51) Int. Cl.: A23L 1/30, A61K 35/74

(54) **Lactobacillus reuteri DSM 17938 for the development of cognitive function**

(71) Applicant: Nestec S.A., 1800 Vevey (CH)
(72) Inventor: Bergonzelli Degonda, Gabriela, 1030 Bussigny (CH); Faure, Magali, 1072 Forel (CH); Kusy, Nicole, 1674 Montet (CH)
(74) Representative: Corticchiato, Olivier

(57) **Abstract**

The invention relates to *Lactobacillus reuteri* DSM 17938 for promoting the establishment of healthy and normal cognitive function in young mammals. In particular, the invention relates to the use of *Lactobacillus reuteri* DSM 17938 for promoting development of the brain structures responsible for cognitive functions (e.g. cortex and hippocampus) and its associated neural pathways and/or to reverse retardation and/or to prevent retardation of the establishment of cognitive functions. Humans or animals and, in particular, a foetus, pre- term or term born infant, toddler or child or a young adult may benefit from the invention.

## Description

### Field of the Invention

The present invention relates generally to the field of neuronal health, neuronal protection and neuronal development. The invention specifically relates to administration of probiotic bacteria capable of promoting the healthy establishment of cognitive function in infants, especially preterm, low birth, very low and extremely low birth weight infants.

### Background to the Invention

The nervous system is a highly complex network composed of neuronal and glial cells. It is present in all mammalian species and is composed of the central nervous system (brain and spinal cord) and the peripheral nervous system (somatic, autonomous and enteric nervous system).

The central nervous system (CNS), and in particular the brain, drives the cognitive functions. The cerebral cortex, which is a sheet of neural tissue that is outermost to the cerebrum of the mammalian brain, plays a key role in attention, perceptual awareness, thought, language, higher order cognition (executive function) and information integration sensory input. It is constituted of up to six horizontal layers, each of which has a different composition in terms of neurons and connectivity. The human cerebral cortex is 2-4 mm (0.08-0.16 inches) thick.

The phylogenetically most recent part of the cerebral cortex, the neocortex (also called isocortex), is differentiated into six horizontal layers; the more ancient part of the brain, the hippocampus (also called archicortex), has, at most, three cellular layers, and is divided into subfields. Neurons in the various layers connect vertically to form small microcircuits, called columns. Different neocortical architectonic fields are distinguished upon variations in the thickness of these layers, their predominant cell type and other factors such as neurochemical markers. [Kandel, E.R., et al. (2000); Principles of Neural Science Fourth Edition, McGraw-Hill, USA, ISBN 0-8385-7701-6, p. 324].

Together with the peripheral nervous system, the CNS has a fundamental role in the control of behaviour. The somatic nervous system is responsible for coordinating the body's movements (under conscious control). The autonomous nervous system maintains homeostasis in the body activities without conscious control (heart rate, etc).

Central nervous system development and maturation is a highly complex biological phenomenon that involves a number of physiological processes including, for example, neuron and glial cell growth and differentiation, neuronal pathfinding and branching, and establishment of inter neuronal communication (nerve signals) via axon growth and neurotransmitter release. Furthermore, although not all axons are myelinated, myelination (an important function of glial cells) is necessary to insulate the electrical signal carried along the axons, thereby ensuring efficient signal transmission, and preventing cross talk between neighbouring nerves. [Baumann, N. and Pham-Dinh, D. (2001); Biology of Oligodendrocyte and Myelin in the Mammalian Central Nervous System, Physiological Reviews, 81 (2): 871-927]; [Deoni, S.C. et al. (2011); Mapping infant brain myelination with magnetic resonance imaging, J. Neurosci., 31(2): 784-91]. This process begins during pregnancy and continues up until and during adolescence/early adulthood (until 20 years old) [Baumann, N. and Pham-Dinh, D. (2001)]; [Benton, D. (2010); Neurodevelopment and neurodegeneration: are there critical stages for nutritional intervention?, Nutr. Rev., 68 Suppl. 1: S6-10].

Neuronal plasticity, which is defined as the ability of the brain to continuously adapt its functionally and structural organization to changing requirements [Nava, E. and Röder, B. (2011); Adaptation and maladaptation insights from brain plasticity, Prog. Brain Res., 191: 177-94]. Review is important in nervous system maturation. It is essential for the correct functioning of the brain and necessary for cognition, learning and memory. Some of the neuronal markers, including proteins and neurotrophic factors required for, or at least, associated with these physiological processes, have been identified in the literature and studied [Huang, E.J. and Reichardt, L. F. (2001); Neurotrophins: Roles in Neuronal Development and Function, Annu. Rev. Neurosci., 24: 677-736]; [Denny, J.B. (2006); Molecular Mechanisms, Biological Actions, and Neuropharmacology of the Growth-Associated Protein GAP43, Current Neuropharmacology, 4: 293-304]; [Baumann, N. and Pham-Dinh, D. (2001)];. [Musumeci, G. and Minichiello, L. (2011); BDNF-TrkB signalling in fear learning: from genetics to neural networks, Rev. Neurosci., 22(3):303-15]; [Xiao, J. et al. (2009); The role of neurotrophins in the regulation of myelin development, Neurosignals, 17: 265-276] and [Von Bohlen and Halbach, O. (2011); Immunohistological markers for proliferative events, gliogenesis, and neurogenesis within the adult hippocampus, Cell Tissue Res., 345(1):1-19].

The central nervous system develops during gestation and then refines to a mature, functional network during the post natal period.

Brain development in humans starts shortly after fertilization of the ovum. The brain develops from the ectoderm layer. In a first step, the neural tube forms. Its bulges give rise to the cortex, thalamus and hypothalamus, midbrain, cerebellum and medulla [Kaufmann, L. *et al.* (2007)]; [Johnson, M. (2005); Kognitive Entwicklungsneuropsychologie, Developmental Cognitive Neuroscience, Göttingen: Hogrefe; (2 ed.) Oxford: Blackwell Publishing]. The brain stem is the posterior part of the brain and provides the main motor and sensory innervation to the face and neck via the cranial nerves. It includes the medulla oblongata (myelencephalon), pons (part of metencephalon), and midbrain (mesencephalon).

In humans foetus, the cerebral cortex develops quite late. After neurogenesis neurons migrate from inner parts (ventricular and subventricular zones) to specific areas of the brain (future cortex). A network of glia cells grows that supports this cell migration.

Premature babies show very basic electrical activity in the primary sensory regions of the cerebral cortex--those areas that perceive touch, vision, and hearing--as well as in primary motor regions of the cerebral cortex. In the last trimester, foetuses are capable of simple forms of learning, like habituating (decreasing their startle response) to a repeated auditory stimulus, such as a loud clap just outside the mother's abdomen. In spite of these rather sophisticated abilities, babies enter the world with a still-primitive cerebral cortex, and it is the gradual maturation of this complex part of the brain that explains much of their emotional and cognitive maturation in the first few years of life [Lubsen, J. et al. (2011); Microstructural and functional connectivity in the developing preterm brain, Seminars in Perinatology, 35, 34-43].

Thus, immaturity or delayed maturation of the CNS, leads to delayed establishment and inadequate functioning of the important biological functions that it regulates. Thus, immaturity or delayed maturation of the cerebral cortex may lead to delayed and/or impaired learning ability, loss of, or poor development of higher reasoning, concentration difficulties, delay in language development, memory and executive function problems, decreased intelligence, and thus, poor mental performance. Other disorders, such as mood disorders and disorders linked to the inability to communicate and socialize normally (for example, autism, including Asperger's syndrome), may also result.

This can be observed in infants such as:
- Preterm infants, low birth weight (<2500 g), very low and extremely low birth weight infants (<1500 g), and in small for gestational age infants [Allen, M.C. (2008); Neurodevelopmental outcomes of preterm infants, Curr. Opin Neurol., 21(2): 123-8].
- Premature or term-born infants having experienced an intrauterine growth retardation (IUGR) that occurred following any adverse events during the gestation (smoking of the mother, medication of the mother, low placenta quality, abnormal placenta positioning, malnutrition of the mother and the foetus, excessive stress/anxiety of the mother, etc); [Gregory, A. et al. (2008); Intrauterine Growth Restriction Affects the Preterm Infant's Hippocampus, Pediatric Research, 63(4): 438-443].
- Any neonate and young infant showing nervous system growth retardation following, for example, hypoxemia-ischemia at birth, or any other adverse event [Barrett, R.D. et al. (2007); Destruction and reconstruction: hypoxia and the developing brain, Birth Defects Res. C. Embryo Today, 81: 163-76].

Thus, if the foetus, neonate or infant has experienced central nervous system growth retardation, it is desirable that this retardation be reversed quickly, if possible, or any further retardation be prevented, so that the central nervous system development "catches up" to a normal level and that the growing foetus or infant experiences as little as possible cognitive function impairment later in life.

It is known that nutrition plays an important role in neuronal maturation in the brain (reviewed in [Huppi, P.S. (2008); Nutrition for the Brain, Pediatric Research, 63(3): 229-231]). Specifically, clinical studies have shown that essential fatty acids, are crucial to ensure foetal and postnatal brain development [Chang, C.Y. et al. (2009); Essential fatty acids and human brain, Acta Neurol. Taiwan, 18(4): 231-41]; [Alessandri, J.M. et al. (2004); Polyunsaturated fatty acids in the central nervous system: evolution of concepts and nutritional implications throughout life, Reprod. Nutr. Dev., 44(6): 509-38].

Early breastfeeding and a higher protein intake has also been shown beneficial to neuronal maturation in infants (reviewed in [Huppi, P.S. (2008)].

The consequences of malnutrition can be irreversible and may include poor cognitive development, educability, and thus future economic productivity. [Horton, R; (2008) The Lancet, Vol. 371, Issue 9608, page 179; [Laus, M.F. et al. (2011); Early postnatal protein-calorie malnutrition and cognition: a review of human and animal studies, Int. J. Environ. Res. Public Health., 8(2): 590-612].

Thus, oral interventions are an appropriate way to positively impact on the development of the nervous system, so as to ensure normal development of cognitive function and mental performance in the preterm or term born neonate, infant, toddler, child or young adult or young animal.

As neuronal maturation starts *in utero,* peri-, as well as postnatal interventions correspond to a promising approach to ensure the healthy development and maturation of the parts of the brain responsible for cognitive function, notably the cerebral cortex and the hippocampus. Interventions during pregnancy/lactation may have considerable advantages in terms of convenience and compliance compared to child-directed interventions.

There is a need to promote and support the healthy establishment of cognitive function, and/or to reverse retardation and/or to prevent further delay of the establishment of cognitive function at the earliest possible stage during gestation, as well as during the early phases of newborn life, when the nervous system is rapidly maturing. The central nervous system and, in particular, parts of the cortex, develop until adolescence (15 years), and the central nervous system adapts throughout life, in particular the cortex and the hippocampus [Baumann, N. and Pham-Dinh, D. (2001)]; [Nosarti, C. et al. (2010); Neurodevelopmental outcomes of preterm birth, Cambridge: Cambridge University Press]. Because of the plasticity of the brain throughout life, an adapted nutritional support all along the lifespan is needed [Benton, D. (2010); Neurodevelopment and neurodegeneration: are there critical stages for nutritional intervention?, Nutr. Rev., 68 Suppl 1:S6-10].

The scientific literature reports on the use of probiotics to impact positively on the health of neonates, and infants, in particular, with respect to reducing inflammation, protecting against infection, and positively impacting on bowel habits and gastrointestinal motility. For a recent general review, see [Dobrogosz, W. J., Peacock, T.J. and Hassan, H. M. (2010); Evolution of the probiotic Concept: From Conception to Validation and acceptance in Medical Science, Advances in Applied microbiology, 72: 1-41].

Human-derived *Lactobacillus reuteri* is considered as an endogenous organism of the human gastrointestinal tract, and is present on the mucosa of the gastric corpus and antrum, duodenum and ileum [Reuter, G. (2001); The Lactobacillus and Bifidobacterium microflora of the human intestine: composition and succession, Curr. Issues Intest. Microbiol, 2: 43-53] and [Valeur, N. et al. (2004); Colonization and immunomodulation by Lactobacillus reuteri ATCC 55730 in the human gastrointestinal tract, Appl. Environ. Microbiol., 70: 1176-1181]. There are reports of *L. reuteri* as a promising therapy for many different conditions including diarrheal disease [Saavedra, J. (2000); Am. J. Gastroenterol, 95: S16-S18], infantile colic [Savino, F. et al. (2007); Lactobacillus reuteri (American Type Culture Collection Strain 55730) versus simethicone in the treatment of infantile colic: a prospective randomized study, Pediatrics, 119: e124-e130], eczema [Abrahamsson, T.R. et al. (2007); Probiotics in prevention of IgE-associated eczema: a double-blind, randomized, placebo-controlled trial, J. Allergy Clin. Immunol., 119: 1174-1180] and H. pylori infection [Imase, K. et al. (2007); Lactobacillus reuteri tablets suppress Helicobacter pylori infection - a double-blind randomised placebo-controlled crossover clinical study, Kansenshogaku Zasshi, 81: 387-393]. A recent study on four *L. reuteri* strains, DSM17938, ATCC PTA4659, ATCC PTA 5289 and ATCC PTA 6475 demonstrated that the strains differentially modulated lipopolysaccharide induced inflammation in small intestinal epithelial cells and in the ileum of newborn rats.

In a double blind randomized study carried out on thirty preterm newborns, reported in 2008 [Indrio, F. et al. (2008); The effects of probiotics on feeding tolerance, bowel habits, and gastrointestinal motility in preterm newborns, J. Pediatr., 152(6): 801-6], the newborns receiving *Lactobacillus reuteri* ATCC 55730 showed a significant decrease in regurgitation and mean daily crying time and a larger number of stools compared with those given placebo.

In a more recent clinical study on 249 preterm newborn babies [Romeo, M.G. et al, (2011); Role of probiotics in the prevention of the enteric colonization by Candida in preterm newborns: incidence of late onset sepsis and neurological outcome, J. Perinatology, 31: 63-69], the babies that were fed *Lactobacillus reuteri* ATCC 55730 (administered for 6 weeks) showed a significant decrease in gastrointestinal colonization by *Candida* species. The reduction of the number of sepsis events was also accompanied by a lower incidence of abnormal neurological outcome, as measured by the Hammersmith Infant Neurological Examination (HINE), for the children receiving the probiotic supplementation. The HINE measures reflexes and functioning of the basic brain structures of the infant - the cerebellum and brain stem. The measurements made in this study are - the cranial nerve function (facial appearance, eye appearance, auditory and visual response, sucking/swallowing), movement, tone, motor milestones, as well as states of consciousness, emotional state and social orientation). The test is a screening method for detecting damage to basic brain functions (for example, as that observed with cerebral palsy). It does not measure concentration, learning, memory or higher order cognition of the infant.

To date, there has been no report on the effect of probiotics on the development of the cognitive functions of the brain. On a physiological level, this translates as the healthy development and maturation of the cerebral cortex and its associated neural pathways that allow efficient transmission of information (from the exterior to the brain, and from the brain to the body) as well as processing of this information within the brain.

Thus, there is a need for a further progress in area of probiotic administration as a way to improve the health of young mammals, including preterm and term-born babies, infants, toddlers and children.

There is, in particular, a need to support the establishment of healthy and normal cognitive function in young mammals, including especially preterm and term-born babies, infants, children and young adults.

There is a need to support the healthy development of learning and memory, attention, perception, thinking, feeling and reasoning in young mammals, including especially preterm and term-born babies, infants and young children.

There is a need to prevent or minimize the severity of cognitive impairment in young mammals. This cognitive impairment may take the form of delayed and/or impaired learning ability, loss of reasoning, memory dysfunction, concentration difficulties (including Attention Deficit Disorder), decreased intelligence, and thus, poor mental performance. This cognitive impairment may also take the form of disorders associated with the inability to normally communicate and socialize, for example, autism, including Asperger's syndrome.

There is a need to positively impact neuronal maturation in the brain of young mammals, in particular, the structures of the brain associated with cognitive function. Specifically, there is a need to positively impact neuronal growth, survival, plasticity and differentiation.

There is a need to positively impact signal transmission in the brain by supporting myelination.

The present invention applies to all mammals, including animals and humans.

### Summary of the invention

The invention relates to *Lactobacillus reuteri* DSM 17938 for promoting the establishment of healthy and normal cognitive function in young mammals. In particular, the invention relates to the use of *Lactobacillus reuteri* DSM 17938 for promoting development of the brain structures responsible for cognitive functions (e.g. cortex and hippocampus) and their associated neural pathways, and/or to reverse retardation and/or to prevent retardation of the establishment of cognitive functions. Humans or animals and, in particular, a foetus, pre- term or term born infant, toddler or child or a young adult may benefit from the invention.

The invention may be especially beneficial to those infants having experienced IUGR, or having a low, very low or extremely low birth weight, being small for gestational age, and/or suffering from cognitive function impairment, such as impaired learning and memory, lack of curiosity, poor attention span and thus, poor mental performance, central nervous system growth retardation, either *in utero,* or, during or after birth.

The present inventors have found that administration of *Lactobacillus reuteri* DSM 17938 promotes neuronal and glial development in young mammals. It ensures healthy neuronal growth, survival, differentiation and plasticity, as well as promoting axon myelination. Administration of *Lactobacillus reuteri* DSM 17938 may increase expression levels of Growth Associated Protein 43 (GAP43), Brain Derived Neurotrophic Factor (BDNF), Glial Fibrillary Acidic Protein (GFAP) or Myelin Basic protein (MBP) in the cortex and/or hippocampus of the young mammal.

*Lactobacillus reuteri* DSM 17938 may be administered to the foetus *in utero,* via the expectant mother

*Lactobacillus reuteri* DSM 17938 may be administered directly to the infant or toddler in its pure form, or diluted in water or breast milk, in a food supplement, or in a milk fortifier, or any enteral feeding, including milk support, during trophic feeding, in an infant formula, or in a milk-based drink.

The *Lactobacillus reuteri* DSM 17938 may be administered to the infant, toddler or child as a daily dose of from 1x10³ to 1x10¹², preferably, 1x10⁷ to 1x10¹¹ cfu (cfu = colony forming unit).

The *Lactobacillus reuteri* DSM 17938 may be administered to the foetus or infant for a duration of at least one week, preferably two weeks, more preferably at least one month. Administration to a toddler or young child may be for at least 4 weeks, preferably 2-12 months, and more preferably for a period of at least 18 months. Administration to an older child or young adult may be up until the age of 15 or even 20 years old.

The invention also relates to a composition comprising *Lactobacillus reuteri* DSM 17938 for promoting the establishment of healthy cognitive function in the brain of young mammals. The invention may be particularly useful for those young mammals who have suffered from IUGR.

### Brief Description of the Drawings

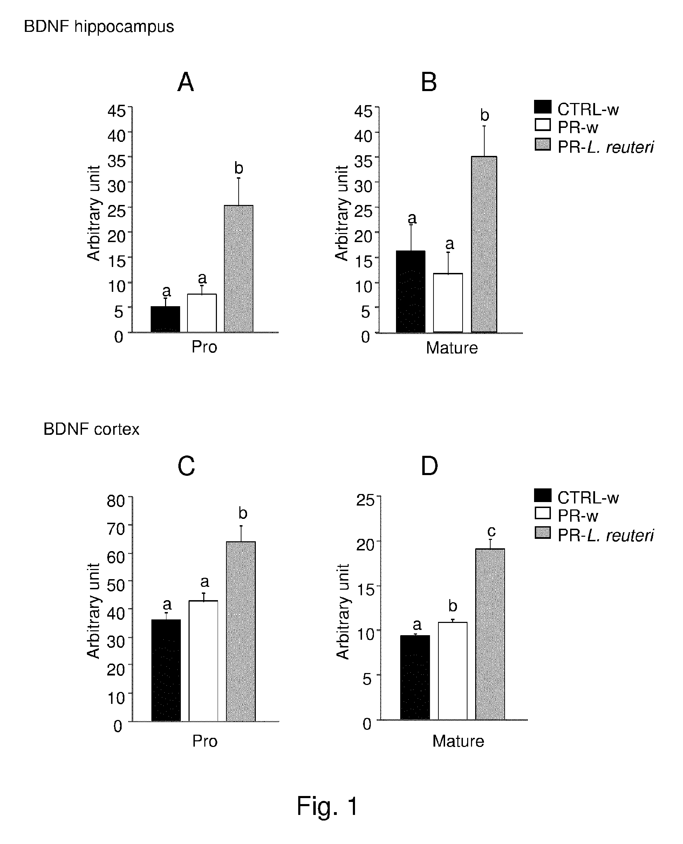

**Figure 1** Pro and mature Brain Derived Neurotrophic Factor (BDNF) protein levels in protein-restricted rat pups supplemented with *L. reuteri* DSM 17938.
   **A, B:** Hippocampus
   Pro **(A)** and mature **(B)** BDNF protein levels in the hippocampus of CTRL-w, PR-w and *PR-L.reuteri* pups at sacrifice (PND14). Results are expressed in arbitrary units, and are medians ± SEMedian, n=6. In each graph, medians without a common letter differ, *P*<0.05. **C,D:** Cortex
   Pro **(C)** and mature **(D)** BDNF protein levels in the cortex of CTRL-w, PR-w and PR-*L.reuteri* pups at sacrifice (PND14). Results are expressed in arbitrary units, and are medians ± SEMedian, n=6. In each graph, medians without a common letter differ, *P*<0.05.
**Figure 2** Growth associated protein 43 (GAP43) protein levels in protein-restricted rat pups supplemented with *L. reuteri* DSM 17938.
   GAP43 protein level in the hippocampus **(A)** and cortex **(B)** of CTRL-w, PR-w and *PR-L.reuteri* pups at sacrifice (PND14). Results are expressed in arbitrary units, and are medians ± SEMedian, n=6. In each graph, medians without a common letter differ, *P*<0.05.
**Figure 3** Glial Fibrillary Acidic Protein (GFAP) protein level in protein-restricted rat pups supplemented with *L. reuteri* DSM 17938.
   GFAP levels were measured in the hippocampus **(A)** and cortex **(B)** of CTRL-w, PR-w and *PR-L.reuteri* pups at sacrifice (PND14). Results are expressed in arbitrary units, and are medians ± SEMedian, n=6. In each graph, medians without a common letter differ, *P*<0.05.
**Figure 4** Myelin Basic protein (MBP) protein levels in protein-restricted rat pups supplemented with *L. reuteri* DSM 17938.
   MBP protein level in the cortex of CTRL-w, PR-w and *PR-L.reuteri* pups at sacrifice (PND14). Results are expressed in arbitrary units, and are medians ± SEMedian, n=6. Medians without a common letter differ, *P*<0.05.

### Detailed description

### Definitions:

In this specification, the following terms have the following meanings:
   *"Probiotic"* means microbial cell preparations or components of microbial cells with a beneficial effect on the health or well-being of the host [Salminen, S. *et al.* (1999);
   Probiotics: how should they be defined, *Trends Food Sci. Technol.,*10: 107-10]. The definition of probiotic is generally admitted and in line with the WHO definition. The probiotic can comprise a unique strain of micro-organism, a mix of various strains and/or a mix of various bacterial species and genera. In case of mixtures, the singular term "probiotic" can still be used to designate the probiotic mixture or preparation. For the purpose of the present invention, micro-organisms of the genus *Lactobacillus* are considered as probiotics.
   *"Prebiotic"* generally means a non digestible food ingredient that beneficially affects the host by selectively stimulating the growth and/or activity of micro-organisms present in the gut of the host, and thus attempts to improve host health.
   *"Pre-term infant"* means an infant born before 37 weeks gestation.
   *"Term Born Infant"* means an infant born after 37 weeks gestation.
   *"Toddler"* means a child from when he can walk up to three years old.
   *"Young child"* means a child from the age of three up to ten years old.
   *"Child"* means up to the age of eighteen years old.
   *"Cognitive Function"* refers to an intellectual process by which one becomes aware of, perceives, or comprehends ideas. It involves all aspects of perception, thinking, reasoning, and remembering. It is distinct from psycho motor function, which is related to reflexes and tone patterns.
   *Lactobaccilus reuteri* DSM 17938, referred to as *L. reuteri* throughout the text, is the *L. reuteri* strain owned by Biogaia AB, Sweden, having the scientific strain designation DSM 17938, formerly *L. reuteri* ATCC 55730. The DSM identification refers to the DSMZ Deutsche Sammlung von Mikroorganismen und Zellkulturen GmbH Inhoffenstr. 7b, D-38124 Braunschweig, Germany. DSM 17938. Deutsche Sammlung von Mikroorganismen und Zellkulturen GmbH Inhoffenstr. 7b D-38124 Braunschweig - Germany.

The present invention provides a probiotic, Lactobacillus reuteri DSM 17938, for the promotion of the healthy establishment of effective cognitive function in a young mammal.

The administration of the probiotic may be to a foetus via the mother. It may also be to a pre-term or term-born infant either directly or via mothers' milk. The administration may be also to a young child, generally up to the age of ten, or to an older child up to the age of fifteen years old, or the equivalent age in an animal. The administration of the *L*. *reuteri* to the young mammal, which may be human (foetus, infant, toddler, child or young infant) or animal, has a positive effect on the healthy establishment of cognitive function, allowing the nervous system associated with these functions to mature and develop normally. This effect is especially beneficial for those who have experienced, for example, intra uterine growth retardation (IUGR) that may have occurred following any adverse event during the gestation (for example, active or passive smoking of the mother, medication of the mother, low placenta quality, abnormal placenta positioning, malnutrition of the mother and/or the foetus, etc).

The present inventors have found that *L*. *reuteri* and/or *L*. *reuteri* containing compositions of the present invention may be used to promote neuronal and glial cell growth, survival, plasticity and differentiation. *L. reuteri* and/or *L. reuteri* containing compositions of the present invention promote axon myelination and neuronal plasticity. Administration of *L. reuteri* and/or *L. reuteri* containing compositions of the present invention thus promotes the establishment of effective cognitive function, including learning and memory, and thus, mental performance. These effects have been demonstrated in an animal model measuring expression levels of proteins that are associated with these biological processes.

Thus, administration of *L. reuteri* and/or *L. reuteri* containing compositions also has benefits for mammals experiencing abnormal cognitive function (cognitive function impairment). Cognitive function impairment may follow, for example, any stress situations, such as those affecting the foetus (*in utero*) such as IUGR, mentioned above, or the newborns (hypoxia-ischemia at birth, oxygen therapy and hyperoxia, inflammation, need for parenteral support, etc), or any cause leading to oxidative stress.

In infants, the *L. reuteri* and/or the *L. reuteri* containing compositions of the present invention may be used to protect the central nervous system, and in particular the structures in the brain associated with establishment of effective cognitive function, i.e. the cerebral cortex and its associated neural pathways, from any stress, e.g., occurring during the neuronal development period, and - consequently - to limit and/or prevent stress-induced neuronal growth retardation and associated cognitive function impairment.

Thus, *L. reuteri* may be administered, in the context of the present invention, when there has been already observed a retardation in the development of the CNS and, in particular, in the brain. Additionally the *L. reuteri* may be administered, prophylactically, when no such retardation has, as yet, been observed.

By administering the *L. reuteri* according to the present invention, the brain, including, in particular, the cerebral cortex and its associated neural pathways, of the treated subject develops healthily. More specifically, the expression of a number of proteins associated with axon myelination, neuronal growth and plasticity (all of which essential for learning and memory are upregulated. Thus, the administration has a positive impact on learning and memory, and thus mental performance. The risk of the infant, or toddler, child or young adult, thus treated, suffering from pathologies associated cognitive function impairment after birth is reduced. This impairment includes delayed and/or impaired learning ability, loss of higher reasoning, learning disabilities, concentration difficulties, decreased intelligence, and thus, poor mental performance. Other disorders linked to the inability to communicate and socialize normally (for example, autism), may also result.

The beneficial effect of *L. reuteri* on the healthy development of the mammalian central nervous system with respect to effective establishment of cognitive function is elaborated upon in the paragraphs below.

### Doses of probiotic:

The probiotic may be administered as a daily dose and in the form of a composition. The daily dose of *L. reuteri* administered to the expectant or breast feeding mother is from 1x10⁶ to 1x10¹² cfu, preferably 1x10⁸ to 1x10¹¹ cfu (cfu = colony forming unit). The daily dose, suitable for newborn babies, ranges from 1x10³ to 1x10¹², preferably, 1x10⁷ to 1x10¹¹ cfu.

Thus, *L. reuteri* may be present in the composition in a wide range of percentages provided that it delivers the beneficial effect described. However, preferably, the *L. reuteri* is present in the composition in an amount equivalent to between 1x10³ and 1x10¹² cfu/g of dry composition. Preferably, for administration to the expectant or lactating mother or the young adult, the probiotic is present in an amount equivalent to between 1x10⁴ to 1x10¹¹ cfu/g of dry composition. The amount of probiotic present per gram of dry composition for administration to the neonates, toddlers and children may be lower, preferably, 1x10⁶ to 1x10⁹, and, of course, the daily doses described above should be respected.

The above doses include the possibilities that the bacteria are live, inactivated or dead, or even present as fragments such as DNA or cell wall materials. In other words, the quantity of bacteria which the formula contains is expressed in terms of the colony forming ability of that quantity of bacteria as if all the bacteria were live irrespective of whether they are, in fact, live, inactivated or dead, fragmented or a mixture of any or all of these states.

### Method of administration:

(i) Administration to expectant mothers:
   The composition can be administered to the expectant mothers by various ways as long as it induces a contact between the composition and the gastro-intestinal tract of the females. Preferably, the composition is orally administered as part of the food, drinks or dietary supplements of the expectant mothers. The composition can also be administered in a pharmaceutical composition. Preferably, the administration is oral. However, in pathological conditions or when enteral feeding is otherwise used, the administration of the composition can be added to the enteral feeding.
(ii) Administration to newborn progeny:
   The *L. reuteri* can also be administered orally, directly to the progeny alone (pure or diluted in water or mother's milk for example) as a supplement (for example as a human milk fortifier supplement), or as a pharmaceutical or nutraceutical composition, or as an ingredient in an infant milk formula. Such a formula may be an infant "preterm formula" if the progeny is born before term or has a low birth weight, a "starter formula" or a "follow-on formula". The formula may also be an hypoallergenic (HA) formula in which the cow milk proteins are hydrolysed. An example of such a starter formula is given in **Example 2.**
(iii) Administration to toddlers, young children and older children, up to the age of about fifteen years old, and young adults up to the age of about twenty years old:
   The *L. reuteri* can also be administered orally to toddlers, children and young adults in the form of a pharmaceutical or nutraceutical composition, growing-up milk, milk-based drinks, food supplements, milk based yoghurts, desserts and puddings, biscuits and cereal bars, cereals and fruit based drinks.
(iv) Administration to animals:
   The *L. reuteri* may also be administered orally to animals alone, or in water, or in the form of a food supplement, a pharmaceutical or nutraceutical composition, or milk or pet food.

### Administration with other compounds:

The *L. reuteri* can be administered alone (pure, or diluted in water or milk, including breast milk, for example) or in a mixture with other compounds (such as dietary supplements, nutritional supplements, medicines, carriers, flavours, digestible or non-digestible ingredients). Vitamins and minerals are examples of typical dietary supplements. In a preferred embodiment, *L. reuteri* is administered in a composition, for example, an infant formula, together with other compounds that enhance the described beneficial effect on the young mammals. Such synergistic compounds may be carriers or a matrix that facilitates the *L. reuteri* delivery to the intestinal tract or they may otherwise enhance the effect of the composition on the enteric nervous system of the progeny. Such compounds can be other active compounds that synergistically or separately influence the development of the enteric nervous system in the infant and/or potentiates the effect of the probiotic. An example of such synergistic compounds is maltodextrin. One of the effect of maltodextrin is to provide a carrier for the probiotic, enhancing its effect, and to prevent aggregation.

Other examples of synergistic compounds that may be included in the compositions, especially infant formula, of the invention are prebiotic compounds. A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon, where they are selectively fermented by the beneficial bacteria. Examples of prebiotics include certain oligosaccharides, such as fructooligosaccharides (FOS), cow milk oligosaccharides (CMOS) and galactooligosaccharides (GOS). A combination of prebiotics may be used such as 90% GOS with 10% short chain fructo-oligosaccharides such as the product sold under the trade mark Raftilose® or 10% inulin such as the product sold under the trade mark Raftiline®. Other examples of prebiotics that can be used in the context of the present invention include the group of oligosaccharides obtained from milk or other sources, optionally containing sialic acid, fructose, fucose, galactose or mannose. Preferred prebiotics are sialo-oligosaccharides (SOS), fructo-oligosaccharides (FOS), galacto-oligosaccharides (GOS), isomalto-oligosaccharides (IMO), xylo-oligosaccharides (XOS), arabino-xylo oligosaccharides (AXOS), mannan oligosaccharides (MOS), oligosaccharides of soy, glycosylsucrose (GS), lactosucrose (LS), sialyl-lactose (SL), fucosyl-lactose (FL), lacto-N-neotetraose (LNNT), lactulose (LA), palatinose-oligosaccharides (PAO), malto-oligosaccharides, gums and/or hydrolysates thereof, pectins, starches, and/or hydrolysates thereof. An infant formula according to the invention preferably further contains at least one prebiotic in an amount of 0.3 to 10% of the total weight of the dry composition.

The daily doses of carbohydrates, and all other compounds administered with the *L. reuteri* should always comply with the published safety guidelines and regulatory requirements. This is particularly important with respect to the administration to newborn babies, especially those born with low birth weight, very low or extremely low birth weight.

The composition, for example infant formula, containing the *L. reuteri* may contain a protein source in an amount of not more than 4.0, 3.0 or 2.0 g/100kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. In one embodiment, the protein content is between 30% and 80% whey proteins. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and betalactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The composition may also comprise a source of carbohydrates and/or a source of fat. The infant formula may contain a source of lipids. The lipid source may be any lipid or fat which is suitable for use in infant formulas. Preferred fat sources include palm olein, high oleic sunflower oil and high oleic safflower oil. The essential fatty acids, linoleic and α-linolenic acid may also be added as small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1 ; for example about 8:1 to about 10:1.

An additional source of carbohydrate may be added to the nutritional composition. It preferably provides about 40% to about 80% of the energy of the nutritional composition. Any suitable carbohydrate may be used, for example sucrose, lactose, glucose, fructose, corn syrup solids, maltodextrin, or a mixture thereof.

Additional dietary fibre may also be added if desired. If added, it preferably comprises up to about 5% of the energy of the nutritional composition. The dietary fibre may be from any suitable origin, including for example soy, pea, oat, pectin, guar gum, acacia gum, fructooligosaccharide or a mixture thereof. Suitable vitamins and minerals may be included in the nutritional composition in an amount to meet the appropriate guidelines.

Examples of minerals, vitamins and other nutrients optionally present in the infant formula include vitamin A, vitamin B1, vitamin B2, vitamin B6, vitamin B 12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

The infant formula may optionally contain other substances which may have a beneficial effect such as fibres, lactoferrin, nucleotides, nucleosides, and the like.

One or more essential long chain fatty acids (LC-PUFAs) may be included in the composition. Examples of LC-PUFAs that may be added are docosahexaenoic acid (DHA) and arachidonic acid (AA). The LC-PUFAs may be added at concentrations so that they constitute greater than 0.01% of the fatty acids present in the composition.

One or more food grade emulsifiers may be included in the nutritional composition if desired; for example diacetyl tartaric acid esters of mono- and di- glycerides, lecithin and mono- or di-glycerides or a mixture thereof. Similarly, suitable salts and/or stabilisers may be included. Flavours can be added to the composition.

### Administration period:

The duration of the administration may vary. While positive effects are expected with relatively short duration of administration (for example, daily administration during one to two weeks for newborns), longer durations are believed to provide an enhanced effect, or, at least, to maintain the effect in older infants (for example, a duration of three, five, eight or 12 months) or in young children (for example, a duration up to the age of 4 or 6 or even 10 years old ). Administration may continue up until the child is about fifteen or even about twenty years old. For administration to animals, the corresponding durations apply.

The expectant mother may start to take the *L. reuteri* as soon as she is aware of her pregnancy. However, the administration period may also start before pregnancy starts, for example if the female is trying to become pregnant. Administration may start at any time after the pregnancy starts. It may start relatively late in the pregnancy, preferably at month 3, 4, 5, 6, 7, 8 or 9 of the pregnancy, in the case of human pregnancy, or in corresponding periods for other mammals, or up to two weeks before the expected delivery date.

The period of administration can be continuous (for example, up to and including lactation up to weaning), or discontinuous. Continuous administration is preferred for a more sustained effect. However, it is speculated that a discontinuous pattern (for example, daily administration during one week per month, or during alternate weeks) can induce positive effects on the progeny.

The administration may cover at least part of the gestation period and at least part of the lactation period if the newborn is fed with mother's milk, or the equivalent period, should the newborn not be fed with mother's milk. Preferably, the administration period to the expectant mother covers substantially the full length of the gestation period, although this may be less. Similarly, the administration period for the lactating mother preferably covers substantially the full length of the lactation period, although, again, this period may be less.

Preferably, the administration to the mother is by daily intake (to be taken once or twice a day), or weekly intake (to be taken one or twice a week).

The *L. reuteri* may be administered to the infant directly. This is the case particularly if the mother does not breastfeed, or after she discontinues breastfeeding. However, an infant who is being breastfed may also receive the *L. reuteri* by direct administration.

Preferably, the administration to the infant is by daily intake. For example, if the *L. reuteri* is administered as an infant formula, the administration is with each feed, i.e. about four to about six times daily for infants less than one year old, the number of feeds reducing with age. For infants older than one year, the administration may be less, once or twice a day. For toddlers and young children the administration may be daily or weekly (to be taken one or twice a week).

The administration to the infant, either via breastfeeding, or by direct administration, or both methods, may be continued up until the age of six months or even one year or longer. Thus, the *L. reuteri* may be administered during lactation, if lactation takes place, or after partial or full weaning. Administration may continue to the infant through the toddler stage and even, up until the age of twenty years old.

### Effect of the L. reuteri administration:

*L. reuteri* administered to neonates promotes the establishment of effective cognitive function. In a rat model experiment, detailed in **Example 1,** the effect of *L. reuteri* administration on neuronal maturation, and specifically in the cerebral cortex and the hippocampus, structures associated with higher cognitive function, was evaluated.

In this experiment, rat pups which had experienced maternal diet induced intrauterine growth retardation (PR group), and pups which had not experienced IUGR (CTRL) were supplemented, from 2 days after birth, with water (controls; namely CTRL-w and PR-w) or *L. reuteri* (*PR-L.reuteri*).

2 weeks after birth, at sacrifice, the neuronal development in the brain was evaluated by the levels of neuronal markers and neurotrophic factors in the cortex and hippocampus.

### Increased expression of Brain Derived Neurotrophic Factor (BDNF):

Brain Derived Neurotrophic Factor (BDNF) is a neurotrophic factor that supports the survival, growth and differentiation of neurons [Huang, E. J. and Reichardt, L. F. (2001)]. There are many reports in the literature linking BDNF with learning and memory [Aguiar, A.S. Jr., et al. (2011) ; Short bouts of mild-intensity physical exercise improve spatial learning and memory in aging rats: Involvement of hippocampal plasticity via AKT, CREB and BDNF signaling, Mech. Ageing Dev.*,* [Epub ahead of print]];

The levels of BDNF (precursor protein (pro) which is then proteolytically cleaved to give the mature form (mature)) were evaluated in the hippocampus and cortex of pups at sacrifice (PND14). The results are shown in **Figure 1****.**

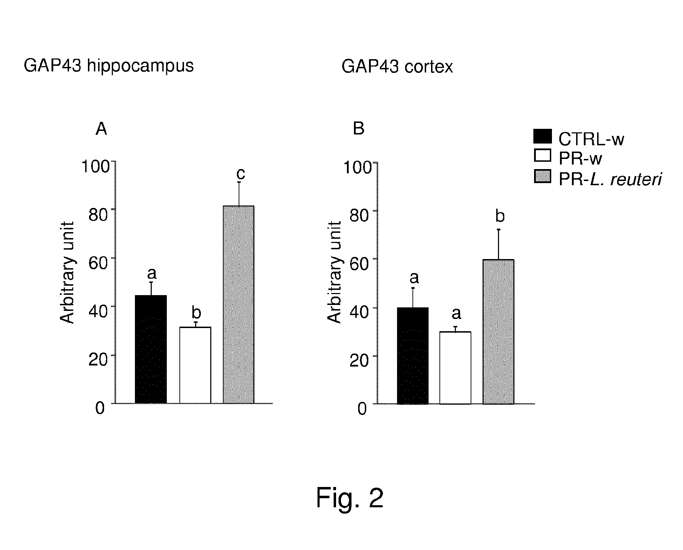

In PR-w pups, the level of pro and mature BDNF was maintained at the CTRL-w level, in the hippocampus **(****Figure 1** **A, B** ), while the level of mature BDNF was slightly increased in the cortex of PR-w pups as compared to CTRL-w **(****Figure 2 B)****.** The supplementation with *L. reuteri* from PND2 to PND14 was accompanied by a significant increase in the levels of pro and mature BDNF, both in hippocampus and cortex.

It has been reported that drug induced improvements in learning and memory dysfunction in rats are associated with increased expression levels of BDNF [Dai, M.H., et al. (2011); Effect of venlafaxine on cognitive function and hippocampal brain-derived neurotrophic factor expression in rats with post-stroke depression, Zhejiang Da XueXueBao Yi Xue Ban, 40(5): 527-34]

Furthermore it has been reported that BDNF is important in hippocampal plasticity, which has a direct effect on learning and memory. [Aguiar, A.S. Jr., *et al.* (2011)].

BDNF expression levels are also important in mood. [Hashimoto, K. (2010); Brain-derived neurotrophic factor as a biomarker for mood disorders: an historical overview and future directions, Psychiatry Clin. Neurosci., 64(4): 341-57]. Review. Erratum in: Psychiatry Clin Neurosci. 2010 Oct;64(5): 590.

Considering these data and the known role of BDNF in neuronal survival, growth and differentiation processes, the increased expression level observed in the current animal model likely translates into biological and cognitive benefits and neuroprotection, during the postnatal development.

### Increased expression of Growth Associated Protein 43 (GAP43):

Growth associated protein (GAP43) is a growth and plasticity protein highly expressed in neuronal growth cones during development [Mercken, M. et al. (1992); Immunocytochemical detection of the growth-associated protein B-50 by newly characterized monoclonal antibodies in human brain and muscle, J. Neurobiol., 23(3): 309-21]. It is a marker for neuronal development. [Denny, J.B. (2006)]. The various interactions specified by the structural domains of the protein are thought to underlie its role in synaptic plasticity, participating in membrane extension during neuritogenesis, in neurotransmitter release and long-term potentiation [Oestreicher, A.B., et al. (1997); B-50, the growth associated protein-43: modulation of cell morphology and communication in the nervous system, Prog. Neurobiol., 53(6): 627-86].

Furthermore, it has been shown that GAP43 is important for memory [Rekart, J.L. et al. (2005); Hippocampal-dependent memory is impaired in heterozygous GAP43 knockout mice, Hippocampus, 15(1): 1-7].

It has been recently reported that isoflurane exposure during pregnancy could cause postnatal spatial memory and learning impairments in offspring rats, which may be partially explained by the down-regulation of GAP43 in the hippocampal area. [Kong, F.J. et al. (2011); Effects of isoflurane exposure during pregnancy on postnatal memory and learning in offspring rats, Mol. Biol. Rep.*,* [Epub ahead of print]].

The protein expression levels of GAP43 were measured in the animal model of **Example 1** and the results are shown in **Figure 2****.** Protein levels were measured in the hippocampus **(****Figure 2A****)** and the cortex **(****Figure 2B****).** The protein level of GAP43 was significantly decreased (P=0.04) in the hippocampus of PR-w pups as compared to CTRL-w (see **Figure 2A****),** while it was not statistically affected in the cortex **(****Figure 2B****).** This reflects a reduced neurogenesis in the hippocampus of PR-w pups as compared to controls.

The supplementation with *L. reuteri* from PND2 to PND14 significantly increased the GAP43 protein level in both brain segments. This suggests the potential of *L. reuteri* to promote neurogenesis in these tissues, during neuronal development, which likely translates into cognitive benefits, later in life. Specifically, given these results, along with what we know about the role of GAP43 in neuronal maturation, we can establish that administration of *L. reuteri* to young mammals favours neuronal growth, survival, differentiation, and plasticity, in the hippocampus and cortex, thus favouring learning and memory and, thus, mental performance.

GAP43 is also important for mood regulation. Recently, it was reported that early parental deprivation in a nonhuman primate, in the absence of subsequent stressors, had a long-term effect on the hippocampal expression of genes implicated in synaptic function and plasticity. The reductions in GAP43 and serotonin 1A receptor expression levels observed in the marmoset monkey model, were comparable with findings in mood disorder. According to the authors, this supported the possibility that the latter mood disorder reflected an early developmental contribution to disease vulnerability [Law, A.J. et al. (2009) Early parental deprivation in the marmoset monkey produces long-term changes in hippocampal expression of genes involved in synaptic plasticity and implicated in mood disorder, Neuropsychopharmacology, May;34(6): 1381-94].

Interestingly, a deficiency in GAP43 has been linked with autistic behaviour. [Zaccaria, K.J., et al. (2010); Resistance to change and vulnerability to stress: autistic-like features of GAP43-deficient mice, Genes Brain Behav., 9(8): 985-96].

Thus, based on this data, one may hypothesize that administration of *L. reuteri,* according to the current invention, may also help prevent and/or attenuate behavioral disorders with a link to GAP 43, such as autism and mood disorders.

### Increased expression of GFAP (Glial Fibrillary Acidic Protein) and MBP (Myelin Basic Protein):

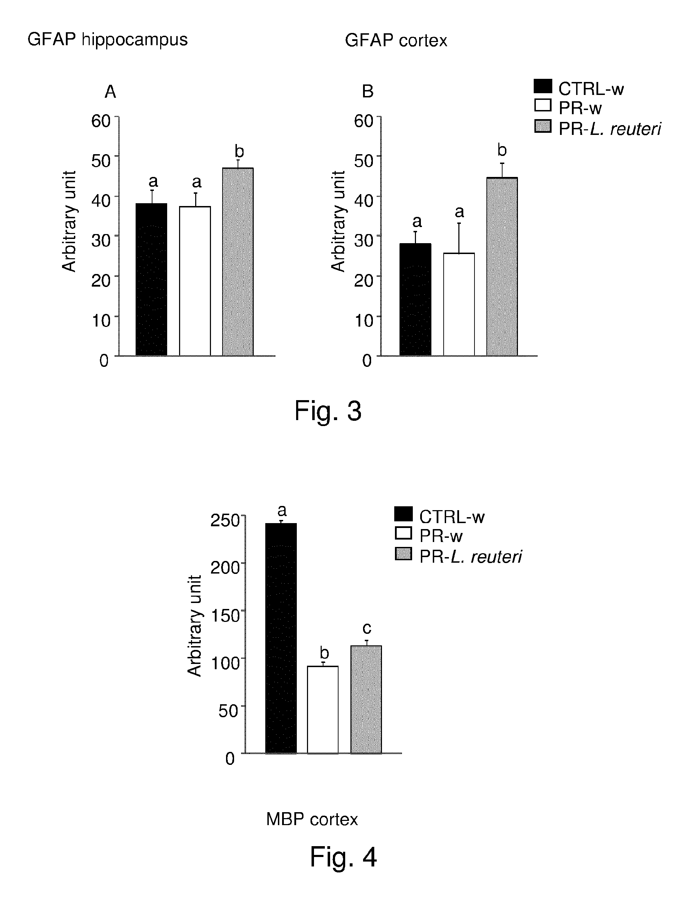

Expression levels of Glial Fibrillary Acidic Protein (GFAP) and Myelin Basic Protein (MBP) were measured in the animal model of **Example 1.** The results are shown in **Figure 3****,** (GFAP, **A** for the hippocampus, and **B** for the cortex) and **Figure 4** (MPB cortex).

Glial Fibrillary Acidic Protein (GFAP) is an intermediate filament protein important for myelination, structure maintenance and for the proper functionning of the blood brain barrier [Eng, L.F. et al. (2000); Glial fibrillary acidic protein: GFAP-thirty-one years (1969-2000), Neurochem. Res., 25(9-10): 1439-51]. A recent report shows that GFAP expression is acutely increased in the early phase of postnatal development [Kim, J.S., et al. (2011); Differential patterns of nestin and glial fibrillary acidic protein expression in mouse hippocampus during postnatal development, J. Vet. Sci., 12(1): 1-6].

Myelin Basic Protein (MBP) is a protein important in the process of myelination of nerves.

Protein levels of GFAP were not affected by the protein restriction in the hippocampus or in the cortex **(****Figure 3****).** It was, however, significantly increased by the *L. reuteri* supplementation in both brain segments. This suggests that administration of *L. reuteri* has a beneficial effect, associated with increased expression levels of GFAP in the hippocampus and cortex, thus ensuring the myelination process, maintaining the neuronal structure, and the proper functioning of the blood brain barrier.

Myelination plays a key role in the developmental phase of the human brain, continuing for at least 10 to 12 years after birth before being completed. [Baumann, N. and Pham-Dinh, D. (2001)]. The rate of development of myelination, therefore, determines the rate of development of related brain functions.

The myelination process is important to convey fast neural signal propagation, allowing effective tissue connectivity within different brain regions, and improving neural pathways connecting separate brain regions required for cognitive, sensory and motor functions.

*L. reuteri* is therefore a new nutritional solution to promote the myelination development process in the brain during the neonatal period.

Such an effect of *L. reuteri* on the myelination process is corroborated by the increased expression of MBP, another protein also involved in the myelination process of nerves **(****Figure 4****).**

The results of the experiments, described in **Example 1,** demonstrate that the administration of *L. reuteri* promotes neuronal growth, survival, differentiation, plasticity in the cortex and hippocampus. Correct neural signaling is ensured via the promotion of myelination and neuron plasticity. Thus, *L. reuteri* administration supports the establishment of cognitive function, in particular, learning and memory, and thus, mental performance.

Administration of *L. reuteri* helps prevent and/or reduce the severity of cognitive function impairment and/ mood deregulation/ disturbances.

The *L. reuteri* strain is a probiotic isolated from the fecal microbiota of a breastfed child. Thus, with respect to a health promoting strategy trying to mimic as closely as possible the microbiota of breastfed children, the administration of *L. reuteri,* particularly to non-breast fed children, may provide an advantage over other strains that are not found in breastfed children.

### Example 1:

### Animal study (feeding and sacrifice) :

Animal experiments were conducted under authorization No. 2120 granted by the Office Vétérinaire Cantonal, Etat de Vaud. Two months old female Sprague-Dawley rats were obtained after one week of gestation from Harlan, Barcelona. On the day of their arrival, rat dams were placed in individual cages and randomly assigned either to control (CTRL) or protein restricted (PR) groups. Animals had access to food and water *ad libitum* and were maintained in a 12 hr light/dark cycle.

Diets of CTRL and PR dams are detailed in Table 1. CTRL dams received a control diet containing 20 % of proteins (casein) fitting standard rat protein requirement during gestation (Reeves *et al.,* 1993). PR dams received a PR diet containing 10% of proteins (casein). Both diets were iso-caloric, the protein deficit being balanced by addition of corn starch.
Diets

**Table 1: Composition of control (CTRL) and protein restricted (PR) AIN-93G diets**

| Components | CTRL | PR |
|---|---|---|
| Cornstarch | 53 | 63 |
| Caseine (K-Caseinate) | 20 | 10 |
| Sucrose | 10 | 10 |
| Soybean oil | 7 | 7 |
| Cellulose | 5 | 5 |
| Mineral mix AIN-93G | 4 | - |
| Mineral mix AIN-93M | - | 4 |
| Vitamin mix AlN-93 | 1 | 1 |
| Choline Bitartrate | 0.25 | 0.25 |
| L-Cysteine | 0.3 | 0.3 |
| Tert-buthylydroquinone | 0.0014 | 0.0014 |

CTRL and PR dams received their respective diets during both gestation and lactation until the day of sacrifice (postnatal day 14 (PND 14)).

On PND 2, pups were randomly assigned to dams from the same experimental group, and litter size was adjusted to 9 pups per dam with a minimal number of four to five males per litter.

From PND 2 till PND14, a daily hand/pipette feeding supplementation of water or *L. reuteri* containing solution was administered to control or treated groups, respectively. The volume of supplementations was gradually adapted to match the growth of rat pups (150 µl/ 100g body-weight).

The groups and diets were as follow:
1) CTRL-w: CTRL pups born from CTRL dams, receiving a supplementation of water,
2) PR-w: PR pups born from PR dams, receiving a supplementation of water,
*3) PR-L.reuteri:* PR pups born from PR dams, receiving a supplementation of *L. reuteri* DSM 17938 freeze-dried (1.10⁹ cfu/day).

At PND 14, a maximum of 10 pups from CTRL and PR groups were weighed and then sacrificed by decapitation after halothane anaesthesia.

### Evaluation of Protein Expression levels:

After isolation, the hippocampus and cortex from PND14 pups of each group were homogenized with a ball beater Tissue Lyser II (Qiagen,USA), in a solution of PBS, pH 7.4, and a complete protease inhibitor cocktail (30mg tissue per 500 µl solution buffer, Roche Diagnostics, Mannheim, Germany). The protein concentration was determined (BCA, Bio Rad). Proteins (20 - 40 micrograms, depending on the marker to assess) were separated by SDS-Page, transferred to nitrocellulose membrane and subsequently blocked in 3% BSA. The relative levels of BDNF (Brain-Derived Neurotrophic Factor), GAP43 (Growth Associated Protein 43), GFAP (Glial Fibrillary Acidic Protein) and MBP (Myelin Basic Protein) were assessed using the specific antibodies Rb α BDNF (Santa Cruz), Mse α GAP43/B50 (Millipore), Mse α GFAP (Cell signaling), and Mse α MBP (Millipore). Detection was made using HRP-chemiluminescence reagents (ECL plus, Amersham), and quantified by using the software AIDA Basic.

### Statistics :

The effect of protein restriction was evaluated by comparing PR with CTRL groups. The effect of the L. reuteri supplementation was evaluated by comparing PR-L. reuteri with PR groups, and an eventual restoration to CTRL levels was evaluated by comparing PR-L. reuteri with CTRL groups. Nonparametric methods were used to analyse the data. Wilcoxon rank sum test was used to test the differences between the treatments. Hodges-Lehmann estimate of the pair-wise treatment difference with its 95% confidence interval was also obtained.

### Example 2:

An example of the composition of an infant formula for use according to the present invention is given below. This composition is given by way of illustration only. The protein source is a conventional mix of whey protein and casein.

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Prebiotic (100% GOS) (g) | 0.64 | 4.3 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| *L. reuteri* | 2x10⁷ cfu/g of powder | |
| DSM 17938 | | |

## Claims

1. *Lactobacillus reuteri* DSM 17938 for promoting the healthy establishment of cognitive function and/or prevention of, or repair of, or reduction in the severity of cognitive function impairment in a young mammal.

2. *Lactobacillus reuteri* DSM 17938 according to claim 1, wherein the young mammal is an animal.

3. *Lactobacillus reuteri* DSM 17938 according to claim 1, wherein the young mammal is a human foetus, pre-term or term born infant, toddler or child or young adult.

4. *Lactobacillus reuteri* DSM 17938 according to any one of claims 1-3, wherein the young mammal has experienced or is experiencing IUGR, and/or had, or is predicted to have, a low, very low, or extremely low birth weight, is small for gestational age, and/or is suffering or suffered from cognitive function impairment either *in utero* or, during, or after birth.

5. *Lactobacillus reuteri* DSM 17938 according to claim 4, wherein the cognitive function impairment is due to hypoxemia-ischemia at birth.

6. *Lactobacillus reuteri* DSM 17938 according to any of the preceding claims, wherein administration to the foetus is *via* the expectant mother.

7. *Lactobacillus reuteri* DSM 17938 according to claim 6, wherein administration to the expectant mother begins when she is 1, 2, 3, 4, 5, 6, 7, 8 or 9 months pregnant, or equivalent time for administration to an expectant animal.

8. *Lactobacillus reuteri* DSM 17938 according to any of claims 1-5, wherein administration to the young mammal is direct or indirect, via the lactating mother.

9. *Lactobacillus reuteri* DSM 17938 according to any of the preceding claims, wherein administration period to the foetus or infant has a duration of at least one week, preferably two weeks, more preferably at least one month.

10. *Lactobacillus reuteri* DSM 17938 according to any of the preceding claims, wherein administration period to the toddler or young child has a duration of at least 4 weeks, preferably 2-12 months, and more preferably for a period of at least 18 months.

11. *Lactobacillus reuteri* DSM 17938 according to any of the preceding claims, wherein administration period for a child is up until the child is 4 years old, preferably, 6 years old, and more preferably up to fifteen years old, and the administration period for a young adult is until early adulthood, approximately 20 years old.

12. *Lactobacillus reuteri* DSM 17938 according to any of the preceding claims, wherein the *Lactobacillus reuteri* DSM 17938 is administered directly to the infant or toddler in its pure form, or diluted in water or breast milk, in a food supplement, or in a milk fortifier, any enteral feeding including milk support during trophic feeding, in an infant formula, or in a milk-based drink.

13. *Lactobacillus reuteri* DSM 17938 according to claim 12, wherein the infant formula is a formula for premature infants, a starter formula or a follow-on formula or a growing-up milk.

14. *Lactobacillus reuteri* DSM 17938 according to any of the preceding claims, wherein administration to the expectant or lactating mother or child or young adult is orally, preferably in foods, drinks, dietary supplements or pharmaceutical compositions.

15. *Lactobacillus reuteri* DSM 17938 according to any of the preceding claims, wherein said *Lactobacillus reuteri* DSM 17938 is administered to infant, toddler or child or young adult as a daily dose of from 1x10³ to 1x10¹², preferably, 1x10⁷ to 1x10¹¹ cfu (cfu = colony forming unit).

16. *Lactobacillus reuteri* DSM 17938 according to any of the preceding claims, wherein said *Lactobacillus reuteri* DSM 17938 is administered to the expectant or lactating mother, or baby, or toddler or child or young adult as a composition comprising between 1x10³ and 1x10¹² cfu/g of dry composition.

17. *Lactobacillus reuteri* DSM 17938 according to claim 16, wherein said composition comprises further ingredients or prebiotics, preferably selected from inulin, fructooligosaccharide (FOS), short-chain fructooligosaccharide (short chain IOS), galacto-oligosaccharide (GOS) and cow milk oligosaccharides (CMOS).

18. *Lactobacillus reuteri* DSM 17938 according to claim 16, or 17 wherein the composition also comprises one or more additional probiotics.

19. *Lactobacillus reuteri* DSM 17938 according to claim 18, wherein the one or more additional probiotic(s) is/are preferably selected from *Bifidobacterium longum* BB536 (ATCC BAA-999), *Lactobacillus rhamnosus* (CGMCC 1.3724), *Bifidobacterium lactis* (NCC2818) or mixtures thereof.

20. *Lactobacillus reuteri* DSM 17938 according to any of the preceding claims, wherein the said *Lactobacillus reuteri* DSM 17938 has been inactivated such as to render it non-replicating.

21. *Lactobacillus reuteri* DSM 17938 according to any of the preceding claims for promoting cognition, learning and/or memory and/or mental performance in a young mammal.

22. *Lactobacillus reuteri* DSM 17938 according to any of the preceding claims for promoting concentration ability, perception, executive function, language ability and mood in a young mammal.

23. *Lactobacillus reuteri* DSM 17938 according to any of claims of the preceding claims for preventing and/or reducing the severity of any one of impaired learning ability, loss of, or poor development of executive functions, memory impairment, concentration difficulties, decreased intelligence, poor mental performance, mood deregulation/disturbance or a disorder associated with to the inability to normally communicate and socialize, such as autism.

24. *Lactobacillus reuteri* DSM 17938 according to any of the preceding claims, for increasing expression levels of any one of GAP43, BDNF, GFAP or MBP in the cortex and/or hippocampus of the young mammal.

25. *Lactobacillus reuteri* DSM 17938 according to any of the preceding claims, for promoting any one of myelination, neuronal growth, neuronal survival, neuronal differentiation, and neuronal plasticity, in the cortex and/or hippocampus of a young mammal.

26. A composition comprising *Lactobacillus reuteri* DSM 17938 for promoting the healthy establishment of cognitive function and/or repair, prevent, or reduce the severity of cognitive function impairment in a young mammal.

27. The composition according to claim 26, wherein the composition is a food supplement, milk fortifier, a starter infant formula, a follow-on infant formula, a growing-up milk, or a milk-based drink.
